# EUROPEAN PATENT APPLICATION

(11) **EP 3 838 240 A1**
(43) Date of publication of application: **23.06.2021**
(21) Application number: 19845897.8
(22) Date of filing: 04.10.2019
(51) Int. Cl.: A61F 13/42, A61F 13/44, A61B 5/00, A61B 5/20, A61F 13/49, G06Q 50/22, G01N 27/02, G08B 21/20

(54) **EXCREMENT AMOUNT MEASUREMENT SYSTEM COMPRISING SMART DIAPER**

(71) Applicant: Monit Corp., Dongjak-gu Seoul 07025 (KR)
(72) Inventor: BAEK, Jaeho, Seoul 03424 (KR); SONG, Jaehoon, Seoul 02167 (KR); LEE, Jaehyun, Gwangju-si Gyeonggi-do 12771 (KR); LIM, Seongcheol, Gwangmyeong-si Gyeonggi-do 14255 (KR); KIM, Juho, Yongin-si Gyeonggi-do 16899 (KR); MOON, Jayoung, Gwangmyeong-si Gyeonggi-do 14306 (KR)
(74) Representative: Frenkel, Matthias Alexander
(86) International application number: PCT/KR2019/013038
(87) International publication number: WO 2021/066233

(57) **Abstract**

A defecation amount measurement system including a smart diaper for determining an amount of defecation includes a defecation amount measurement sensing unit including at least one temperature sensor, at least one humidity sensor, at least one gas sensor, and at least one capacitance sensor, a wearing and posture sensing unit including the at least one capacitance sensor, at least one gyro sensor, at least one magnetic field sensor, and at least one acceleration sensor, a transmission unit, which transmits a first sensing information generated by the defecation amount measurement sensing unit and a second sensing information generated by the wearing and posture sensing unit to a determination unit, and a determination unit which receives the first sensing information from the defecation amount measurement sensing unit, receives the second sensing information from the wearing and posture sensing unit, distinguishes a type of the defecation depending on a predetermined threshold value, and determines an amount of the defecation.

## Description

### [TECHNICAL FIELD]

The present invention relates to a defecation amount measurement system including a smart diaper, and more particularly, to a defecation amount measurement system which distinguishes a type of defecation and measures an amount of the defecation.

### [BACKGROUND ART]

Infants, the mobility impaired, and elderly seniors are classified as socially disadvantaged, who always need someone's help for defecation. In reality, however, it is not easy for those in need of help to discharge defecation and urination with someone's help 24 hours a day.

In the case of the defecation, a cycle is irregular and difficult to be predicted due to differences in personal eating habits, and thus the infants, the mobility impaired, the elderly seniors, and the like overcome a difficult situation by wearing diapers.

However, even when defecation using the diaper is not properly managed, skin diseases such as biting or inflammation of a human body may occur and mental atrophy such as lack of self-feeling may be followed.

To this end, Korea Patent No. 10-1881103 is intended to solve through a technology related to a smart infant defecation system.

As a prior document, the smart infant defecation system determines a type of defecation through various sensors and informs timing of diaper replacement. However, the prior art document could not determine an amount of the defecation, but only determine presence of the defecation to unnecessarily send a diaper replacement signal in spite of a small amount of the defecation.

As a result, in order to overcome the above problems, there is a need for a diaper to monitor the amount of the defecation.

### [DETAILED DESCRIPTION OF THE INVENTION]

### [TECHINICAL PROBLEM]

An object of the present invention is to a defecation amount measurement system including a smart diaper.

### [TECHNICAL SOLUTION]

A defecation amount measurement system including a smart diaper for determining an amount of defecation according to an embodiment of the present invention includes a defecation amount measurement sensing unit including at least one temperature sensor, at least one humidity sensor, at least one gas sensor, and at least one capacitance sensor, a wearing and posture sensing unit including the at least one capacitance sensor, at least one gyro sensor, at least one magnetic field sensor, and at least one acceleration sensor, a transmission unit which transmits a first sensing information generated by the defecation amount measurement sensing unit and a second sensing information generated by the wearing and posture sensing unit to a determination unit, and a determination unit which receives the first sensing information from the defecation amount measurement sensing unit, receives the second sensing information from the wearing and posture sensing unit, distinguishes a type of the defecation depending on a predetermined threshold value, and determines an amount of the defecation

In addition, the defecation amount measurement sensing unit may measure temperature, humidity, and concentration of gas of the outside air within/from the surface of diaper when the diaper is worn and transmit the temperature, humidity, and concentration of gas to the determination unit.

Furthermore, the first sensing information is time-series information of temperature, humidity, gas, and capacitance, and the second sensing information may be time-series information of capacitance, angular velocity, magnetic field, and acceleration.

In addition, the determination unit may include a wearing detection module which determines whether to wear by the capacitance sensor, a threshold calculation module which measures the temperature and humidity of the outside air by a predetermined time unit and calculates threshold values of temperature and humidity for each time unit, a defecation determination module which determines whether the temperature and humidity based on the first sensing information exceed the threshold values and determines type and presence of the defecation using information of a gas sensor, a defecation amount prediction module which predicts the amount of the defecation using the first sensing information and the second sensing information, and a communication module which transmits a diaper replacement signal using a first data generated by the defecation determination module and a second data generated by the defecation amount prediction module.

In addition, the defecation amount prediction module may predict an amount of urine in consideration of an amount of change in capacitance and a degree of inclination based on the time-series capacitance information included in the first sensing information and the time-series angular velocity information included in the second sensing information.

Furthermore, the threshold calculation module may be changed by the time unit based on wearer condition, weather and diaper storage condition.

In addition, the defecation amount measurement system may further include an inference unit which stores the first data and the second data generated by the determination unit and performs learning based on artificial intelligence.

### [ADVANTAGEOUS EFFECTS OF THE INVENTION]

A defecation amount measurement system according to an embodiment of the present invention senses a type of defecation and informs the replacement timing of a diaper.

In addition, a defecation amount measurement system according to an embodiment of the present invention measures an amount of the defecation to prevent unnecessary replacement of the diaper or unnecessary labor for replacement.

Furthermore, operating conditions are set actively and automatically depending on the external environmental conditions.

### [DESCRIPTION OF THE DRAWINGS]

FIG. 1 is a functional block diagram of a defecation amount measurement system according to an embodiment of the present invention.
FIG. 2 is an inner view of a smart diaper according to an embodiment of the present invention.
FIG. 3 is a detailed functional block diagram of a determination unit according to an embodiment of the present invention.
FIG. 4 is a view illustrating an electric field change of a capacitance sensor before and after wearing a diaper according to an embodiment of the present invention.
FIG. 5 is a view illustrating an electric field change of a capacitance sensor before and after wearing a diaper according to another embodiment of the present invention.
FIG. 6 is a graph illustrating temperature and humidity measurement data according to an embodiment of the present invention.
FIG. 7 is a view of a diaper wearer lying in bed.
FIG. 8 is a view illustrating another example of a capacitance sensor included in a defecation amount measurement sensing unit according to an embodiment of the present invention.

### [BEST MODE FOR CARRYING OUT THE INVENTION]

The features and effects of the present invention will be more readily apparent from the detailed descriptions below provided in conjunction with the accompanying drawings, allowing the person of ordinary skill in the field of art to which the present invention pertains to readily practice the technical spirit of the invention.

As the present invention allows for various changes and numerous embodiments, particular embodiments will be illustrated in the drawings and described in detail in the written text. However, this is not to limit the present invention to particular modes of practice, and it is to be appreciated that all changes, equivalents, and substitutes that do not depart from the spirit and technical scope of the present invention are encompassed in the present invention.

In describing the drawings, similar reference numerals are designated to similar elements.

While such terms as "first" and "second," etc., may be used to describe various components, such components must not be limited to the above terms. The above terms are used only to distinguish one component from another.

For example, a first component may be referred to as a second component without departing from the scope of rights of the present invention, and likewise a second component may be referred to as a first component. The term "and/or" encompasses both combinations of the multiple number of related items disclosed and any item from among the multiple related items disclosed.

Unless otherwise defined, all terms used herein, including technical or scientific terms, have the same meanings as those generally understood by the person having ordinary skill in the field of art to which the present invention pertains.

Such terms as those defined in a generally used dictionary are to be interpreted to have the meanings equal to the contextual meanings in the relevant field of art, and are not to be interpreted to have ideal or excessively formal meanings unless clearly defined in the present specification.

The terms "module," "block," and "part," used in the descriptions below to denote certain components, may be assigned and/or interchanged for the sake of convenience in presenting the written description, and one such term does not in itself signify a particular meaning or function.

Hereinafter, certain preferred embodiments of the present invention are described below with reference to the accompanying drawings to allow the person having ordinary skill in the art to readily practice the invention. In describing the embodiments of the present invention, certain detailed explanations of the related art are omitted when it is deemed that they may unnecessarily obscure the essence of the invention.

Hereinafter, a defecation amount measurement system including a smart diaper according to the present invention will be described in detail with reference to the drawings.

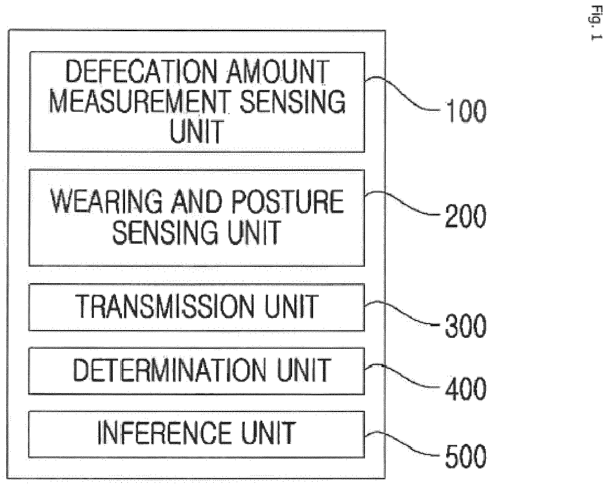

FIG. 1 is a functional block diagram of a defecation amount measurement system according to an embodiment of the present invention.

As illustrated in FIG. 1, a defecation amount measurement system according to an embodiment of the present invention relates to a system for predicting defecation amount of a user wearing a smart diaper and includes a defecation amount measurement sensing unit 100 including at least one gas sensor and at least one capacitance sensor, a wearing and posture sensing unit 200 including the at least one capacitance sensor, at least one gyro sensor, at least one magnetic field sensor, and at least one acceleration sensor, a transmission unit 300 for transmitting a first sensing information generated by the defecation amount measurement sensing unit 100 and a second sensing information generated by the wearing and posture sensing unit 200 to a determination unit 400, and the determination unit 400 receiving the first sensing information from the defecation amount measurement sensing unit 100 and the second sensing information from the wearing and posture sensing unit 200 to distinguish a type of the defecation by a predetermined threshold value and to determine an amount of the defecation.

The defecation amount measurement sensing unit 100 is embedded in a smart diaper to obtain data for determining the type of the defecation and the amount of the defecation. The defecation amount measurement sensing unit 100 includes at least one temperature sensor, at least one humidity sensor, at least one gas sensor, and at least one capacitance sensor.

The defecation amount measurement sensing unit 100 collects information on temperature, humidity, and gas (VOC detection) in the diaper or determines the amount of the defecation by the capacitance sensor. In addition, information on the external environment of the smart diaper, that is, temperature, humidity, and gas may be periodically collected by the temperature and humidity sensors equipped to be exposed to an outer surface of the diaper.

At least one of the temperature sensor, humidity sensor, gas sensor, and capacitance sensor included in the defecation amount measurement sensing unit 100 may be embedded in the diaper and each sensor may be embedded at a position corresponding to each other.

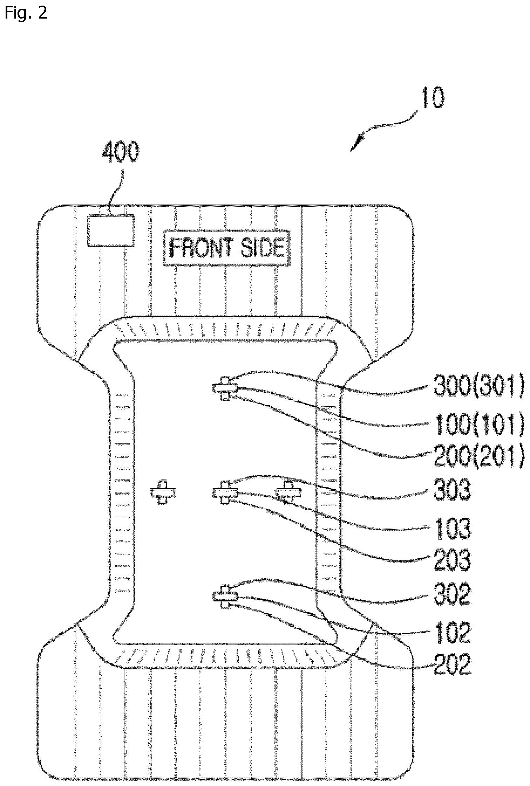

FIG. 2 is an inner view of a smart diaper according to an embodiment of the present invention.

As illustrated in FIG. 2, in the defecation amount measurement system including a smart diaper 10 according to an embodiment of the present invention, various sensors embedded in the smart diaper 10 may be configured as one sensor set and a plurality of sensor sets may be equipped to collect information of various areas. Preferably, the defecation amount measurement sensing unit 100, the wearing and posture sensing unit 200, and the transmission unit 300 may constitute a single sensor set.

For example, the temperature sensor, the humidity sensor, the gas sensor, and the capacitance sensor may be configured as an integrated device. In addition, at least one integrated device may be embedded in an excretion area in which actual excretion is made and may be equipped at symmetrical positions or in areas where excretion is predominant.

The wearing and posture sensing unit 200 may include at least one capacitance sensor, at least one gyro sensor, at least one magnetic field sensor, and at least one acceleration sensor.

The wearing and posture sensing unit 200 generates the second sensing information through the sensors therein and transmits the second sensing information to the determination unit 400 through the transmission unit 300 to be described below.

The wearing and posture sensing unit 200 generates the second sensing information, which is capable of determining whether the user wears the diaper, the posture, or whether the user falls, to transmit the second sensing information to the determination unit 400. In particular, the second sensing information may track a path in which the defecation is absorbed and moves depending of the posture of the wearer. Therefore, the amount of the defecation may be predicted depending on a change of the first sensing information individually equipped in each area.

The second sensing information includes a time-series information such as capacitance, angular velocity, magnetic field, and acceleration.

As described above, the capacitance sensor included in the defecation amount measurement sensing unit 100 and the capacitance sensor included in the wearing and posture sensing unit 200 may be the same, or may be different from each other to derive a separate embodiment.

The transmission unit 300 transmits the first sensing information generated by the defecation measurement sensing unit 100 and the second sensing information generated by the wearing and posture sensing unit 200 to the determination unit 400 to be described below. The transmission unit 300 may continuously transmit the first sensing information and the second sensing information, which are time-series information, to the determination unit 400 or may transmit them by a predetermined period.

The transmission unit 300 may be a communication module capable of near filed communication and may be applied to a wireless communication module having a small size, which is capable of being connected to the determination unit 400.

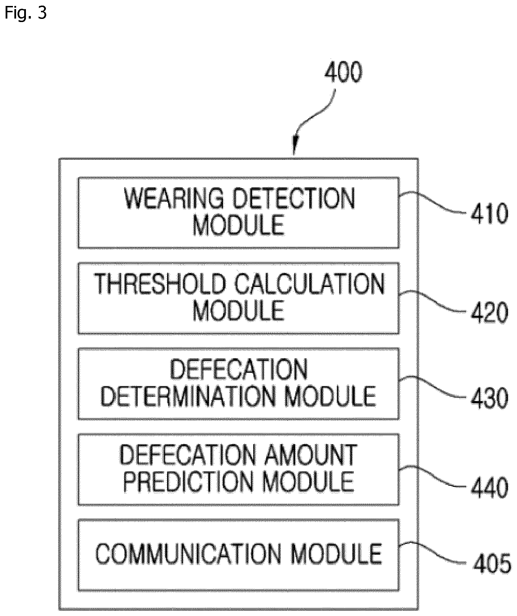

Next, the determination unit 400, which receives the first sensing information and the second sensing information described above and determines the type of the defecation, the amount of the defecation, and the like, will be described in detail with reference to FIG. 3.

FIG. 3 is a detailed functional block diagram of a determination unit according to an embodiment of the present invention.

As illustrated in FIG. 3, the determination unit according to an embodiment of the present invention includes a wearing detection module 410 which determines whether to wear by the capacitance sensor, a threshold calculation module 420 which measures temperature and humidity of the outside air by a predetermined time unit to calculate threshold values of temperature and humidity for each time unit, a defecation determination module 430 which determines whether the temperature and humidity exceed the threshold values based on the first sensing information and determines the type and presence of the defecation using information of the gas sensor, a defecation amount prediction module 440 for predicting the amount of the defecation using the first sensing information and the second sensing information, and a communication module 450 for transmitting a diaper replacement signal using a first data generated in the defecation determination module and a second data generated in the defecation amount prediction module.

The determination unit 400 according to an embodiment of the present invention may be embedded in the smart diaper as illustrated in FIG. 2, or may be implemented as a device adjacent to the smart diaper.

The wearing detection module 410 is a module which determines whether the user wears the diaper. When the actual user wears the diaper, it is importance to detect wearing time because the temperature and humidity of the outside air within/from the surface of diaper should be measured at the beginning of the wearing.

In particular, the temperature and humidity of the outside air at the beginning of the wearing are used to set reference threshold values for determining the presence of the defecation as well as the amount of the defecation.

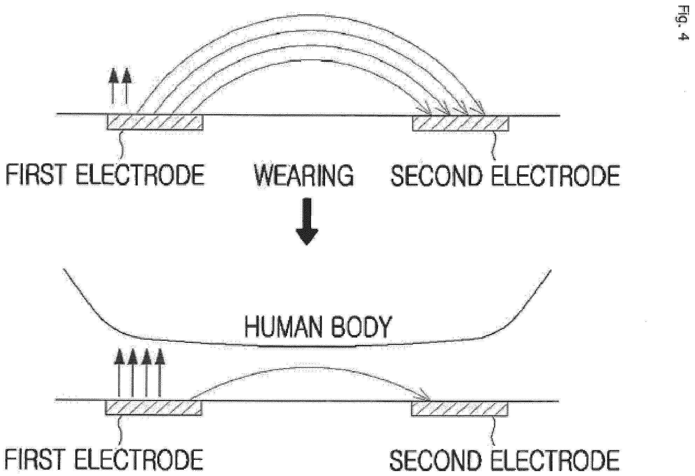

In actual, a method of detecting wearing of the diaper in the wearing detection module 410 will be described with reference to FIG. 4.

FIG. 4 is a view illustrating an electric field change of a capacitance sensor before and after wearing a diaper according to an embodiment of the present invention.

As illustrated in FIG. 4, two electrodes (a first electrode and a second electrode) are equipped at the same layer and the first electrode generates an electric field before wearing the diaper. The electric field generated from the first electrode is directed to the second electrode, which is a ground electrode, and there is a leaking electric field not directed to the second electrode.

Then, when the user wears the diaper, the electric field formed between the first electrode and the second electrode is deformed into another form, a human body becomes a ground electrode similar to the second electrode, and the electric field directed to the second electrode is changed to be directed to the human body who wears the diaper. FIG. 4 illustrates a physical change of the capacitance sensor, which results in a change in capacitance "C" value of the capacitance sensor.

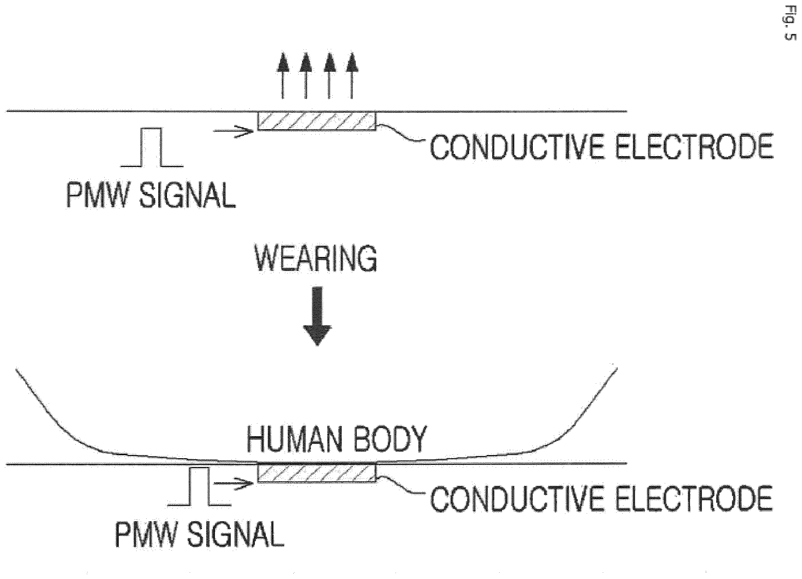

FIG. 5 is a view illustrating an electric field change of a capacitance sensor before and after wearing a diaper according to another embodiment of the present invention.

As illustrated in FIG. 5, the capacitance sensor according to another embodiment of the present invention, which is one conductive electrode, detects a change in capacitance. When a pulse width modulation (PWM) signal is applied to one conductive electrode as illustrated in FIG. 5, free electrons arranged on a surface of the electrode generate an electric field on the surface of the electrode by the PWM signal.

Here, when wearing the smart diaper, the human body acts as a ground electrode and the free electrons arranged on the conductive electrode surface are discharged to the human body not to form the electric filed even by the PMW signal, thereby detecting whether to wear.

The threshold calculation module 420 measures the temperature and humidity of the outside air included in the first sensing information by the predetermined time unit to calculate the threshold value for each time unit. In addition, the threshold values for temperature and humidity may be changed in consideration of a situation of the user, such as a case where the user using the diaper generates heat, a case where a body temperature of the user is high, or the like.

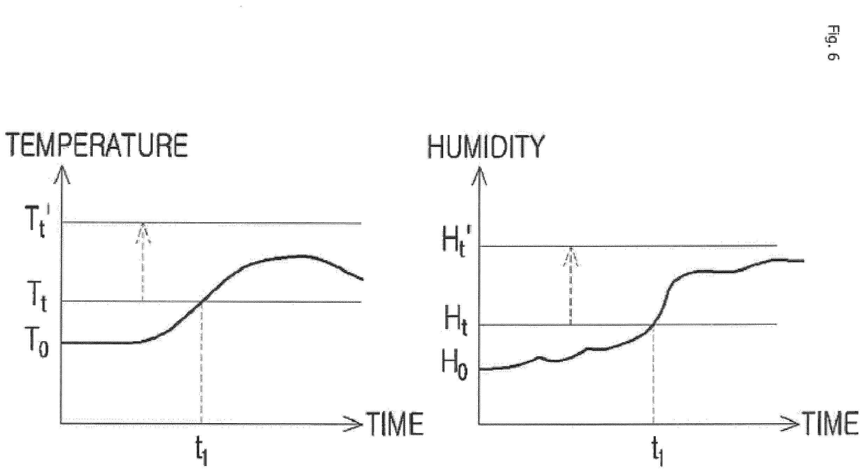

FIG. 6 is a graph of temperature and humidity measurement data according to an embodiment of the present invention.

As illustrated in FIG. 6, a temperature T₀ and a humidity H₀ of the outside air measured at the beginning of wearing are used as main factors for setting an initial threshold temperature value Tₜ and an initial threshold humidity value Hₜ. In addition, the threshold temperature value and the threshold humidity value may be changed by the predetermined time unit depending on a condition of wearer, a weather change, diaper state and condition, and the like. In actually, Tₜ' and Hₜ' represent the threshold values changed depending on the conditions.

For example, as illustrated in FIG. 6, it may be determined that the defecation is performed in the diaper when the temperature and humidity exceed the threshold values at time t₁. However, when the temperature and humidity increase in consideration of the temperature and humidity of the outside air, environment of the user, or the like within a time period t₀ to t₁, it is not determined that there is the defecation.

The change of the threshold value is performed by periodically checking the temperature and humidity of the outside air, and the change of the threshold values is most important in accurately determining the presence of the defecation and the amount of the defecation.

The defecation determination module 430 determines whether the type of the defecation is urine or stool through the information of the temperature sensor, humidity sensor, and gas sensor embedded in the diaper and determines the presence of the defecation through whether the threshold temperature and the threshold humidity are exceed or not.

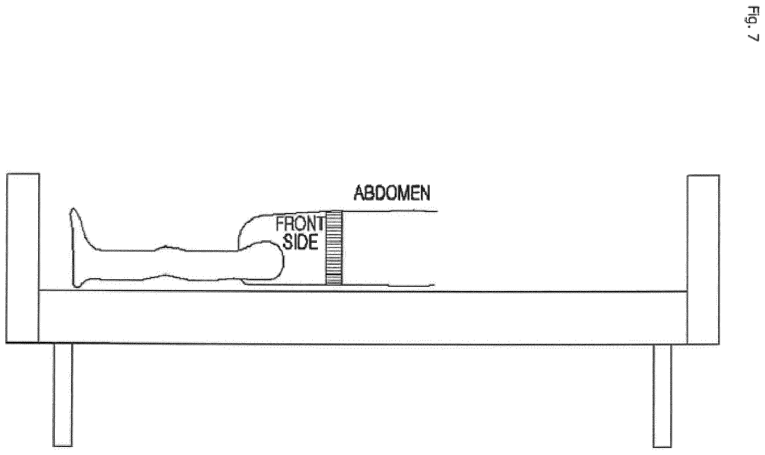

The defecation amount prediction module 440 predicts the amount of the defecation using the first sensing information and the second sensing information measured above. An Example of prediction methods of the defecation amount prediction module 440 will be described with reference to FIGS. 2 and 7.

Fig. 7 is a view of a diaper wearer lying in bed.

The defecation amount prediction module 440 may predict the amount of urine in consideration of a change amount of the capacitance and a degree of inclination using the time-series capacitance information included in the first sensing information and the time-series angular velocity information included in the second sensing information.

When the user sets a situation lying in bed as illustrated in FIG. 7, the stool having relatively low fluidity may be easily determined using the sensing information generated in a defecation amount sensing unit 102 and a wearing and posture sensing unit 202, which are disposed at a rear side of the diaper.

In a case of the urine having high fluidity, the defecation amount prediction module 440 distinguishes large / medium / small defecation in consideration of a position of a body part where the urine is discharged and the degree of inclination of the diaper.

In the situation lying in the bed, the acceleration sensor and gyro sensor in the wearing and posture sensing unit 200 predicts the posture of the user. The inclination and posture of the diaper may be predicted through roll, pitch, and yaw values measured by the gyro sensor. However, the gyro sensor causes an error due to temperature, the error accumulates, and thus a final value is caused to drift.

Therefore, the gyro sensor compensates for the error using the temperature sensor included in the first sensing information. In addition, when it comes to a long time of a stationary state, an inclined angle calculated by the acceleration sensor shows a correct value, but the gyro sensor shows an incorrect value as time passes.

On the contrary, when it comes to a short time of movement, the gyro sensor may show the correct value but the acceleration sensor may have different calculation values than the inclined angle. Thus, the defecation amount prediction module calculates the roll, pitch, and yaw values using an algorithm which is capable of compensating for errors using the acceleration sensor and the gyro sensor to infer the posture of the user.

In addition, the magnetic field sensor may calculate the roll, pitch, and yaw values calculated by the gyro sensor, set a center of reference of the movement using the acceleration sensor, and perform correction of a central axis for each value. Finally, the posture of user is inferred using the gyro sensor, the magnetic field sensor, and the acceleration sensor.

As a result, the posture of the user in the lying state is inferred as illustrated in FIG. 7 and the amount of the defecation is predicted by combining information such as the temperature, humidity, and capacitance in the sensor set equipped at the individual position.

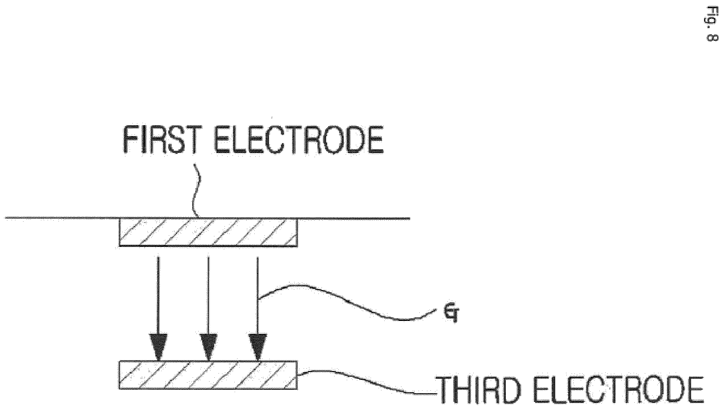

FIG. 8 is a view illustrating another example of a capacitance sensor included in a defecation amount measurement sensing unit according to an embodiment of the present invention.

As illustrated in FIG. 8, the capacitance sensor included in the defecation amount measurement sensing unit 100 predicts the amount of the defecation. According to still another embodiment of the present invention, unlike the capacitance sensor included in the wearing detection module described above with reference to FIG. 4 or 5, the capacitance sensor for defecation amount measurement may be a capacitance sensor having a stacked structure.

In this case, a dielectric formed between the first electrode and a third electrode has a certain dielectric constant. When liquid foreign matter such as urine or watery stool penetrates into the dielectric, the dielectric constant is changed.

In particular, the dielectric constant may vary depending on a height of the liquid foreign material penetrating into the dielectric, changing capacitance "C" may be observed, and thus the amount of the defecation may be predicted.

For example, in the lying state as illustrated in FIG. 7, when a sensing value of a defecation amount measurement sensing unit 101 disposed at a position where the urine is discharged, results in discharging the defecation and a sensing value of the defecation amount measurement sensing unit 103 disposed at the middle of the diaper does not exceed the threshold value not to discharge the defecation, the amount of the defecation may be determined as small (little) or medium (average).

On the contrary, although it is shown that there is no defecation in the defecation amount measurement sensing unit 101 disposed at the position where the urine through the capacitance sensor or the like, the amount of the defecation may be determined as large (much) when the urine is founded to be excreted through the defecation amount measurement sensing unit 103 disposed in the middle of the diaper, the temperature sensor, the humidity sensor, and the capacitance sensor of the defecation amount measurement sensing unit 102 disposed behind.

The communication module 450 transmits the diaper replacement signal to a terminal of an external manager using the first data generated from the defecation determination module 430, i.e., a data related to the type and presence of the defecation, and the second data generated from the defecation amount prediction unit 440, i.e., a data related to large / medium / small of the defecation.

The external manager may recognize the diaper replacement signal through the terminal and replace the diaper of the user at correct timing.

In addition, the defecation amount measurement system including the smart diaper according to an embodiment of the present invention may further include an inference unit 500, which stores the first data and the second data generated by the determination unit described above, performs learning based on artificial intelligence, and accurately infers the presence and amount of the defecation through the accumulated data.

As a result, the defecation amount measurement system including the smart diaper according to an embodiment of the present invention may predict the type, presence, and amount of the defecation using the sensing information obtained from the smart diaper, and ultimately may infer and predict health status, life pattern, and the like of the user based on the defecation.

While the inventive concept has been described with reference to exemplary embodiments, it will be apparent to those skilled in the art that various changes and modifications may be made without departing from the spirit and scope of the inventive concept. Therefore, it should be understood that the above embodiments are not limiting, but illustrative.

## Claims

1. A defecation amount measurement system including a smart diaper for determining an amount of defecation, the system comprising:
a defecation amount measurement sensing unit including at least one temperature sensor, at least one humidity sensor, at least one gas sensor, and at least one capacitance sensor;
a wearing and posture sensing unit including the at least one capacitance sensor, at least one gyro sensor, at least one magnetic field sensor, and at least one acceleration sensor;
a transmission unit configured to transmit a first sensing information generated by the defecation amount measurement sensing unit and a second sensing information generated by the wearing and posture sensing unit to a determination unit; and
the determination unit configured to receive the first sensing information from the defecation amount measurement sensing unit, receive the second sensing information from the wearing and posture sensing unit, distinguish a type of the defecation depending on a predetermined threshold value, and determine an amount of the defecation.

2. The system of claim 1, wherein the defecation amount measurement sensing unit measures temperature, humidity, and concentration of gas of the outside air within/from the surface of diaper when the diaper is worn and transmits the temperature, humidity, and concentration of gas to the determination unit.

3. The system of claim 2, wherein the first sensing information is time-series information of temperature, humidity, gas, and capacitance, and the second sensing information is time-series information of capacitance, angular velocity, magnetic field, and acceleration.

4. The system of claim 2, wherein the determination unit includes:
a wearing detection module configured to determine whether to wear by the capacitance sensor;
a threshold calculation module configured to measure the temperature and humidity of the outside air by a predetermined time unit and calculate threshold values of temperature and humidity for each time unit;
a defecation determination module configured to determine whether the temperature and humidity based on the first sensing information exceed the threshold values and determine type and presence of the defecation using information of a gas sensor;
a defecation amount prediction module configured to predict the amount of the defecation using the first sensing information and the second sensing information; and
a communication module configured to transmit a diaper replacement signal using a first data generated by the defecation determination module and a second data generated by the defecation amount prediction module.

5. The system of claim 4, wherein the defecation amount prediction module predicts an amount of urine in consideration of an amount of change in capacitance and a degree of inclination based on the time-series capacitance information included in the first sensing information and the time-series angular velocity information included in the second sensing information.

6. The system of claim 4, wherein the threshold calculation module is changed by the time unit based on wearer condition, weather and diaper storage condition.

7. The system of claim 4, further comprising:
an inference unit configured to store the first data and the second data generated by the determination unit and perform learning based on artificial intelligence.
